(19) Europäisches Patentamt
European Patent Office
Office européen des brevets

(11) Veröffentlichungsnummer: **0 015 365 A1**

(12) # EUROPÄISCHE PATENTANMELDUNG

(21) Anmeldenummer: **80100205.6**

(22) Anmeldetag: **16.01.80**

(51) Int. Cl.³: **C 07 D 239/42**, C 07 D 239/46, C 07 D 239/34, C 07 D 239/38, C 07 D 239/56, D 06 P 1/66

(30) Priorität: **27.01.79 DE 2903133**

(43) Veröffentlichungstag der Anmeldung: **17.09.80 Patentblatt 80/19**

(84) Benannte Vertragsstaaten: **CH DE FR GB**

(71) Anmelder: **BAYER AG, Zentralbereich Patente, Marken und Lizenzen, D-5090 Leverkusen 1, Bayerwerk (DE)**

(72) Erfinder: **Berger-Lohr, Dr., Heymannstrasse 63, D-5090 Leverkusen (DE)**
Erfinder: **Schündehütte, Karl Heinz, Dr., Klief 75, D-5090 Leverkusen (DE)**
Erfinder: **Söll, Manfred, Dr., Dillinger Strasse 23, D-5090 Leverkusen (DE)**

(54) **Quartäre Reaktivverbindungen, ihre Herstellung und ihre Verwendung zur Erhöhung der Affinität von anionischen Farbstoffen.**

(57) Quartäre Verbindungen der Formel

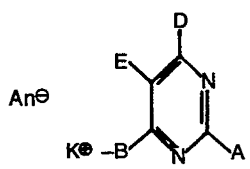

worin

A einen reaktionsfähigen, unter den für Reaktivfarbstoffe üblichen Färbebedingungen abspaltbaren Substituent bedeutet,

und die übrigen Symbole die im Text angegebene Bedeutung haben, werden zur Erhöhung der Affinität von anionischen Farbstoffen zu natürlichen oder synthetischen stickstoff- oder hydroxygruppenhaltigen Fasermaterialien verwendet.

EP 0 015 365 A1

0015365

BAYER AKTIENGESELLSCHAFT 5090 Leverkusen, Bayerwerk
Zentralbereich Mi/Th
Patente, Marken und Lizenzen

Quartäre Reaktivverbindungen, ihre Herstellung und ihre Verwendung zur Erhöhung der Affinität von anionischen Farbstoffen

Die vorliegende Erfindung betrifft quartäre Verbindungen der Formel

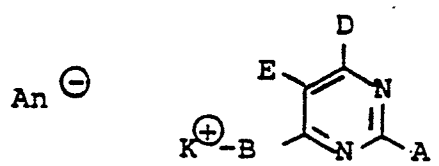

(I)

worin

A ein reaktionsfähiger, abspaltbarer Substituent,

E Wasserstoff, Halogen, gegebenenfalls substituiertes Alkyl, Aryl, Cyano, Aminocarbonyl, Mono- oder Dialkylaminocarbonyl, Alkoxycarbonyl,

Nitro, gegebenenfalls substituiertes Alkyl-, Aryl- oder Aralkylsulfonyl,

D Wasserstoff, Halogen, gegebenenfalls substituiertes Alkyl, Aryl, Aralkyl, Aminocarbonyl, Mono- oder Dialkylaminocarbonyl, Hydroxy, gegebenenfalls substituiertes Alkoxy, Aryloxy, Aralkoxy, Alkylthio, Arylthio, Aralkylthio, gegebenenfalls substituiertes Alkyl-, Aryl- oder Aralkylsulfonyl oder einen Rest der Formel

$$-\underset{G_1}{\underset{|}{N}}-G$$

G und $G_1$ unabhängig voneinander Wasserstoff, gegebenenfalls substituiertes Alkyl, Aryl, Aralkyl oder gemeinsam mit N einen 5- oder 6-gliedrigen Ring bedeuten, worin

A auch für Wasserstoff oder einen nichtreaktionsfähigen Substituenten stehen kann, wenn

D für Halogen oder gegebenenfalls substituiertes Alkyl, Aryl- oder Aralkylsulfonyl steht, und worin

B ein Bindeglied

$$- X - Y - Z - Y_1 -$$

bedeutet,
wobei X mit dem Diazinrest verbunden ist,

X Sauerstoff, Schwefel oder 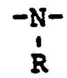,

R Wasserstoff, gegebenenfalls substituiertes Alkyl, Alkenyl oder Aralkyl,

Y 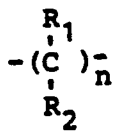,

n die Zahlen 0 bis 4

Z einen gegebenenfalls substituierten Phenylen- oder Naphthylen-Rest oder den Rest der Formel $-CH=CH-$,

$Y_1$ einen Rest der Formeln

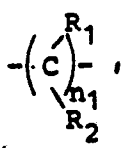

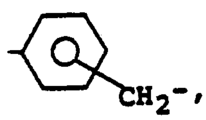

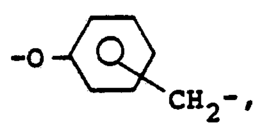

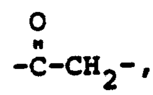

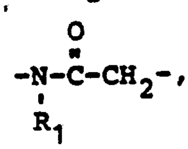

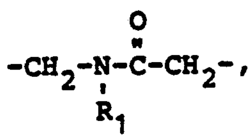

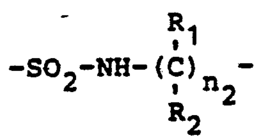

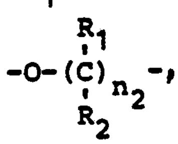

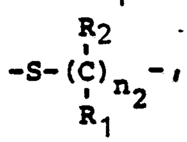

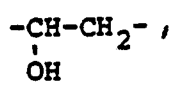

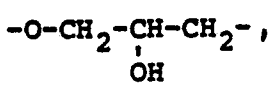

$n_1$ die Zahlen 0 bis 4, wobei n und $n_1$ nicht gleichzeitig 0 sind,

$n_2$ die Zahlen 2 bis 5,

$R_1$ und $R_2$ unabhängig voneinander Wasserstoff oder Alkyl,

$K^{(+)}$ einen quartären Rest

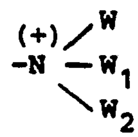

$W, W_1$ und $W_2$ unabhängig voneinander gegebenenfalls substituiertes Alkyl, Cycloalkyl, Alkenyl oder Aralkyl,

$W_1$ und $W_2$ oder

$W, W_1$ und $W_2$ gemeinsam mit dem Stickstoffatom einen gegebenenfalls substituierten Heterocyclus oder

$W$ einen Amino-, Monoalkyl- oder Dialkylamino-Rest oder einen Rest der Formel

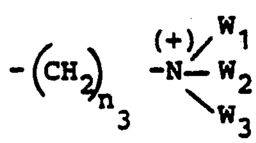

$n_3$ die Zahlen 2 bis 4,

$W_3$ einen gegebenenfalls substituierten Alkylrest und

$An^{(-)}$ ein Anion bedeuten,

ihre Herstellung und ihre Verwendung zur Erhöhung der Affinität von anionischen Farbstoffen zu natürlichen oder synthetischen stickstoff- oder hydroxylgruppenhaltigen Fasermaterialien.

Die Alkyl- und Alkoxyreste haben insbesondere 1-4 Kohlenstoffatome. Sie können durch nichtionische Substituenten, z.B. durch Halogen wie Fluor, Chlor oder Brom oder Cyan oder im Fall von D, G, R, W und $W_3$ durch einen Rest der Formel

$$-\left(O-\overset{R_3}{\overset{|}{C}H}-\overset{R_3}{\overset{|}{C}H}\right)_{n_4}-O-R_4$$

II

worin

$R_3$ beide für Wasserstoff oder 1 $R_3$ für Methyl und das andere für Wasserstoff,

$R_4$ für Wasserstoff, $C_1$-$C_4$-Alkyl, Benzyl, Acetyl, Propionyl oder Benzoyl und

$n_4$ für 0 - 4 stehen,

substituiert sein.

Bevorzugte Arylreste sind Phenylreste und als Aralkylreste sind Benzyl und Phenylethyl bevorzugt. Cycloalkyl steht insbesondere für Cyclopentyl oder Cyclohexyl. Die genannten Ringe können z.B. durch Halogen wie Fluor, Chlor oder Brom, $C_1$-$C_4$-Alkyl, $C_1$-$C_4$-Alkoxy oder Hydroxy substituiert sein.

Alkenyl steht vorzugsweise für Reste mit 2-4 Kohlenstoffatomen.

Die Reste $W_1$ und $W_2$ können gemeinsam mit den Stickstoffatomen z.B. einen 5- oder 6-gliedrigen Ring wie Pyrrolidin-, Piperidin-, Morpholin- oder Piperazinring bilden. Die Reste W, $W_1$ und $W_2$ können gemeinsam mit dem Stickstoffatom z.B. einen Pyridinring bilden. Bevorzugte Substituenten dieser Ringe sind $C_1$-$C_4$-Alkylreste.

Insbesondere ist unter X eine Gruppe der Formel

$$-\underset{R}{\underset{|}{N}}-$$

zu verstehen, in der R für Wasserstoff oder $C_1$-$C_4$-Alkyl, insbesonere aber für Wasserstoff oder Methyl steht.

Als Beispiele für Y sind neben der direkten Bindung eine Methylen-, Ethylen-, Propylen- oder Butylen-Gruppe, insbesondere aber eine Methylen-Gruppe oder die direkte Bindung zu nennen.

Als Beispiel für Z steht ein Rest der Formel -CH=CH-, oder ein durch Fluor, Chlor, Brom, Hydroxy-, $C_1$-$C_4$-Alkyl- oder $C_1$-$C_4$-Alkoxy-Gruppen substituierter Phenylen- oder Naphthylen-Rest, insbesondere aber der unsubstituierte oder durch 1 oder 2 Methyl-Gruppen substituierte Phenylen-Rest.

Von den Bindegliedern $Y_1$ seien hier die Bindeglieder

$$-\left(\begin{matrix}R_1\\|\\C\\|\\R_2\end{matrix}\right)_{n_1}- \;,\quad -SO_2NH-\left(\begin{matrix}R_1\\|\\C\\|\\R_2\end{matrix}\right)_{n_2} \;,\quad -O-\left(\begin{matrix}R_1\\|\\C\\|\\R_2\end{matrix}\right)_{n_2}- \;,\quad -\overset{O}{\overset{\|}{C}}-CH_2-\ \text{und}$$

$$-CH_2-\overset{R_1}{\overset{|}{N}}-\overset{O}{\overset{\|}{C}}-CH_2-,\quad -\underset{OH}{\underset{|}{CH}}-CH_2-,\qquad -O-CH_2-\underset{OH}{\underset{|}{CH}}-CH_2-$$

mit $R_1$ und $R_2$ für Wasserstoff oder $C_1$-$C_4$-Alkyl-Gruppen und $n_1$ für die Zahlen 1 oder 2 und $n_2$ für 2 oder 3 besonders genannt. Insbesondere aber steht $Y_1$ für Methylen oder Äthylen.

Als Beispiele für D sind hervorzuheben: Wasserstoff, Halogen, Methyl, Ethyl, Methoxy, Ethoxy, Methyl-, Ethyl-, Phenyl- und Benzylthio, Methyl-, Ethyl- oder Phenylsulfonyl, Trifluormethyl, gegebenenfalls durch Halogen, Cyan oder Hydroxy substituiertes $C_1$-$C_4$-Alkoxy oder eine Gruppe der Formel II oder eine Gruppe der Formel

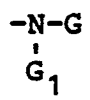

mit G, $G_1$ = Wasserstoff, Methyl, Ethyl, Hydroxyethyl, Phenyl oder Benzyl.

Beispiele für E sind insbesondere: Wasserstoff, Halogen, Methyl, Ethyl, Phenyl, Cyano, Nitro oder Methyl-, Ethyl- oder Phenylsulfonyl.

Unter den reaktionsfähigen, abspaltbaren Substituenten A werden Substituenten verstanden, die unter den für Reaktivfarbstoffe üblichen Färbebedingungen abspaltbar sind. Besonders sind zu erwähnen: Halogen wie Fluor, Chlor oder Brom, Ammonium, Hydrazinium, Sulfonium, gegebenenfalls substituiertes Alkylsulfonyl, Arylsulfonyl, Aralkylsulfonyl, Azido ($N_3$), Aryloxy, Rhodano, Arylthio und Sulfonsäure, wobei besonders Halogen und hier insbesondere Fluor und Chlor oder Methyl-, Ethyl- und Phenylsulfonyl bevorzugt sind.

Entsprechend werden unter nichtreaktionsfähigen Substituenten A solche verstanden, die unter den für Reaktivfarbstoffe üblichen Färbebedingungen nicht abspaltbar sind.

A kann beispielsweise auch für Wasserstoff, Alkyl oder Aryl stehen, wenn D für Halogen, Methyl-, Ethyl- oder Phenylsulfonyl steht.

Halogen bedeutet im Rahmen dieser Erfindung insbesondere Fluor, Chlor oder Brom.

Als Anionen $An^{(-)}$ kommen sowohl Anionen anorganischer wie auch organischer Säuren in Betracht.
Als Beispiele seien genannt: Chlorid, Fluorid, Bromid, Sulfat, Phosphat, Tetrafluoroborat, Anionen aromatischer und niederer aliphatischer Carbon- und Schwefelsäuren wie Benzolsulfonat, p-Toluolsulfonat, Methansulfonat, Äthansulfonat, Methosulfat, Ethosulfat oder Acetat.

Das Anion ist im allgemeinen durch das Herstellungsverfahren und die Isolierung der Produkte (I) gegeben.
Die Anionen können jedoch in bekannter Weise gegen beliebige andere Anionen ausgetauscht werden.

Im einzelnen sind beispielsweise als Pyrimidinylreste zu nennen: Mono-, Di- oder Trihalogenpyrimidinylreste, wie 2,4-Dichlorpyrimidinyl-6-, 2, 4, 5-Trichlorpyrimidinyl-6-, 2,4-Dichlor-5-nitro- oder -5-methyl- oder -5-carboxymethyl- oder -5-carboxy- oder -5-cyano- oder -5-vinyl- oder 5-sulfo- oder -5-mono- -di- oder -trichlormethyl- oder -5-carboalkoxy-pyrimidinyl-6-, sowie die entsprechenden Brom- und Fluor-Derivate der oben erwähnten chlorsubstituierten heterocyclischen Reste, unter diesen beispielsweise 2-Fluor-4-pyrimidinyl-, 2,6-Difluor-4-pyrimidinyl-, 2,6-Difluor-5-chlor-4-pyrimidinyl-, 2-Fluor-5,6-dichlor-4-pyrimidinyl-, 2,6-Difluor-5-methyl-4-pyrimidinyl-, 2,5-Difluor-6-methyl-4-pyrimidinyl-, 2-Fluor-5-methyl-6-chlor-4-pyrimidinyl-, 2-Fluor-5-nitro-6-chlor-4-pyrimidinyl-, 5-Brom-2-fluor-4-pyrimidinyl-, 2-Fluor-5-cyan-4-pyrimidinyl-, 2-Fluor-5-methyl-4-pyrimidinyl-, 2,5,6-Trifluor-4-pyrimidinyl-, 5-Chlor-6-chlormethyl-2-fluor-4-pyrimidinyl-, 2,6-Difluor-5-brom-4-pyrimidinyl-, 2-Fluor-5-brom-6-methyl-4-pyrimidinyl-, 2-Fluor-5-brom-6-chlormethyl-4-pyrimidinyl-, 2,6-Difluor-5-chlor-methyl-4-pyrimidinyl-, 2,6-Difluor-5-nitro-4-pyrimidinyl-, 2-Fluor-6-methyl-4-pyrimidinyl-, 2-Fluor-5-chlor-6-methyl-4-pyrimidinyl-, 2-Fluor-5-chlor-4-pyrimidinyl-, 2-Fluor-6-chlor-4-pyrimidinyl-, 6-Trifluormethyl-5-chlor-2-fluor-4-pyrimidinyl-, 6-Trifluormethyl-2-fluor-4-pyrimidinyl-, 2-Fluor-5-nitro-4-pyrimidinyl-, 2-Fluor-5-trifluormethyl-4-pyrimidinyl-,

2-Fluor-5-phenyl- oder -5-methyl-sulfonyl-4-pyrimidinyl-, 2-Fluor-5-carbonamido-4-pyrimidinyl-, 2-Fluor-5-carbomethoxy-4-pyrimidinyl-, 2-Fluor-5-brom-6-trifluormethyl-4-pyrimidinyl-, 2-Fluor-6-carbonamido-4-pyrimidinyl-, 2-Fluor-6-carbomethoxy-4-pyrimidinyl-, 2-Fluor-6-phenyl-4-pyrimidinyl-, 2-Fluor-6-cyan-4-pyrimidinyl-, 2,6-Difluor-5-methylsulfonyl-4-pyrimidinyl-, 2-Fluor-5-sulfonamido-4-sulfonamido-4-pyrimidinyl-, 2-Fluor-5-chlor-6-carbomethoxy-4-pyrimidinyl-, 2,6-Difluor-5-trifluormethyl-4-pyrimidinyl-, sulfonylgruppenhaltige Pyrimidinringe, wie 2-Car-

boxymethylsulfonyl-pyrimidinyl-4-, 2-Methylsulfonyl-6-methyl-pyrimidinyl-4-, 2-Methyl-sulfonyl-6-äthyl-pyrimidinyl-4-, 2-Phenylsulfonyl-5-chlor-6-methyl-pyrimidinyl-4-, 2,6-Bis-methylsulfonyl-pyrimidinyl-4-, 2,6-Bis-methylsulfonyl-5-chlor-pyrimidinyl-4-, 2,4-Bis-methylsulfonyl-pyrimidin-5-sulfonyl, 2-Methylsulfonylpyrimidinyl-4-, 2-Phenyl-sulfonyl-pyrimidinyl-4-, 2-Trichlormethylsulfonyl-6-methyl-pyrimidinyl-4-, 2-Methylsulfonyl-5-chlor-6-methyl-pyrimidinyl-4-, 2-Methylsulfonyl-5-brom-6-methyl- pyrimidinyl-4-, 2-Methyl-sulfonyl-5-chlor-6-äthyl-pyrimidinyl-4-, 2-Methylsulfonyl-5-chlor-6-chlormethyl-pyrimidinyl-4-, 2-Methylsulfonyl-4-chlor-6-methyl-pyrimidin-5-sulfonyl-, 2-Methylsulfonyl-5-nitro-6-methyl-pyrimidinyl-4-, 2,5,6-Tris-methyl-sulfonyl-pyrimidinyl-4-, 2-Methylsulfonyl-5,6-dimethyl-pyrimidinyl-4-, 2-Äthylsulfonyl-5-chlor-6-methyl-pyrimidinyl-4-, 2-Methylsulfonyl-6-chlor-pyrimidinyl-4-, 2,6-Bis-methylsulfonyl-5-chlor-pyrimidinyl-4-, 2-Methylsulfonyl-6-carboxy-pyrimidinyl-4-, 2-Methylsulfonyl-5-sulfo-pyrimidinyl-4-, 2-Methylsulfonyl-6-carbomethoxy-pyrimidinyl-4-, 2-Methylsulfonyl-5-carboxy-pyrimidinyl-4-, 2-Methylsulfonyl-5-cyan-6-methoxy-pyrimidinyl-4-, 2-Methylsulfonyl-5-chlor-pyrimidinyl-4-, 2-Sulfoäthyl-sulfonyl-6-methyl-pyrimidinyl-4-, 2-Methyl-sulfonyl-5-brom-pyrimidinyl-4-, 2-Phenylsulfonyl-5-chlor-pyrimidinyl-4-, 2-Carboxymethylsulfonyl-5-chlor-6-methyl-pyrimidinyl-4-.

Von den Verbindungen der Formel I sind die der Formel

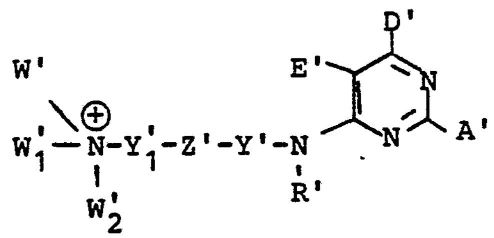

An$^{\ominus}$ (III)

hervorzuheben, worin

A' für Fluor, Chlor, Methyl-, Ethyl- oder Phenylsulfonyl,

E' für Wasserstoff, Fluor, Chlor, Methyl, Ethyl, Phenyl, Cyano, Nitro oder Methyl-, Ethyl- oder Phenylsulfonyl

D' für Wasserstoff, Fluor, Chlor, Methyl, Ethyl, Methyl-, Ethyl- oder Phenylsulfonyl, gegebenenfalls durch Fluor, Chlor, Brom, Cyan oder Hydroxy substituiertes $C_1$-$C_4$-Alkyl, $C_1$-$C_4$-Alkoxy oder $C_1$-$C_4$-Alkylthio oder für einen Rest

$$-\underset{\substack{|\\ G'_1}}{N}-G'$$

mit G' und $G'_1$ = Wasserstoff, gegebenenfalls durch Hydroxyl substituiertes $C_1$-$C_4$-Alkyl, Phenyl oder Benzyl,

oder für

$$-(CH_2)_{n_4}-(O-\overset{\substack{R_3\\ |}}{C}H-\overset{\substack{R_3\\ |}}{C}H)_{n_4}-O-R'_4$$

mit $n_4$ = unabhängig voneinander = 0 bis 4
$R_3$ = beide Wasserstoff oder 1 $R_3$ Wasserstoff und das andere Methyl,
$R_4'$ = Wasserstoff oder $C_1$-$C_4$-Alkyl,

stehen, worin

A' auch für Wasserstoff stehen kann, wenn
D' für Fluor, Chlor, Methyl-, Ethyl- oder Phenylsulfonyl steht, und worin
R' für Wasserstoff oder $C_1$-$C_4$-Alkyl, das durch

$$-(O-\overset{R_3}{\overset{|}{C}H}-\overset{R_3}{\overset{|}{C}H})_{n_4}-O-R_4$$

substituiert sein kann,

Y' für $-\left(\begin{matrix}R_1'\\ | \\ C\\ | \\ R_2'\end{matrix}\right)_{n'}-$

mit $R_1'$ und $R_2'$ = Wasserstoff oder $C_1$-$C_4$-Alkyl,
$n'$ = 0 oder 1,

Z' für gegebenenfalls durch Fluor, Chlor, Brom, Hydroxy, $C_1$-$C_4$-Alkyl oder $C_1$-$C_4$-Alkoxy substituiertes Phenylen oder Naphthylen,
$Y_1'$ für einen Rest der Formeln

$$-\left(\overset{R_1'}{\underset{R_2'}{C}}\right)_{n_1'}- , \quad -\overset{O}{\overset{\|}{C}}-CH_2- , \quad -CH_2-\underset{R_1'}{N}-\overset{O}{\overset{\|}{C}}-CH_2- ,$$

$$-O-(\overset{R_1'}{\underset{R_2'}{C}})_{n_2'}- , \quad -\underset{OH}{CH}-CH_2- \text{ oder } -O-CH_2-\underset{OH}{CH}-CH_2- ,$$

mit

$n_1'$ = 1 oder 2,

$n_2'$ = 2 oder 3,

W', $W_1'$ und $W_2'$ unabhängig voneinander für $C_1$-$C_6$-Alkyl, das durch

$$-(O-\overset{R_3}{CH}-\overset{R_3}{CH})_{n_4}-O-R_4$$

substituiert sein kann,

W' außerdem für Benzyl, Amino, $C_1$-$C_4$-Alkylamino oder $C_1$-$C_4$-Dialkylamino oder

$W_1'$ und $W_2'$ gemeinsam mit dem Stickstoffatom für Pyrrolidin, Piperidin, Morpholin oder Piperazin oder

W', $W_1'$ und $W_2'$ gemeinsam mit dem Stickstoffatom für Pyridin und

$An^{(-)}$ für ein Anion stehen.

Von diesen Verbindungen werden solche mit

n' = null

Z' = gegebenenfalls durch Methyl substituiertes Phenylen und

$Y_1'$ = Methylen oder Ethylen

bevorzugt.

Besonders bevorzugte Verbindungen der Formel (III) sind die der Formel

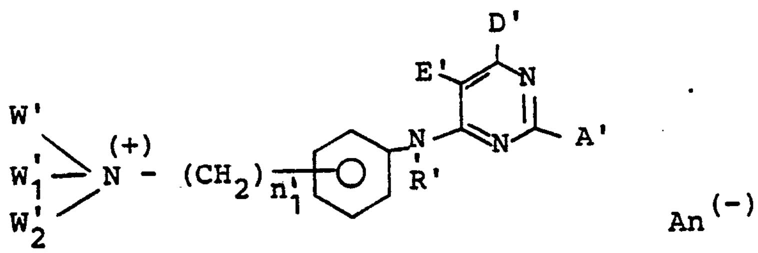

(IV)

worin

A', D', E', R', W', $W_1'$, $W_2'$, $n_1'$ und $An^{(-)}$ die in Formel (III) angegebene Bedeutung haben.

Von diesen Verbindungen der Formel (IV) werden wiederum die mit

R' = Wasserstoff oder Methyl,
W', $W_1'$ und $W_2'$ = Methyl, Ethyl oder Hydroxyäthyl
$n_1'$ = 1

besonders bevorzugt.

Die Herstellung der Verbindungen der Formel (I) kann nach an sich bekannten Methoden z.B. nach den Verfahren 1 und 2 erfolgen:

1. 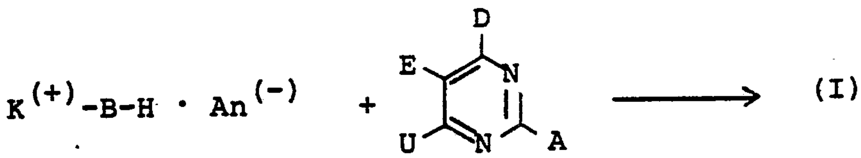

$K^{(+)}$-B-H · $An^{(-)}$ + (I)

(V) (VI)

wobei U ein unter den Reaktionsbedingungen abspaltbarer Rest, insbesondere Halogen oder gegebenenfalls substituiertes Sulfonyl ist, der unter den Reaktionsbedingungen mit z.B. (V) unter Bildung von HU abgespalten wird.

Die Umsetzung kann in wäßriger oder wäßrig-organischer Lösung, vorzugsweise in neutraler oder schwach saurer Lösung bei Temperaturen zwischen 0 und 90°C, vorzugsweise 0-60°C, erfolgen. Die während der Reaktion entstehende Säure HU wird beispielsweise mit Bicarbonat, Soda oder Natronlauge neutralisiert. Die Verbindungen der Formel I werden als feste Substanzen oder in Pastenform aus der Reaktionslösung isoliert. Es ist jedoch nicht erforderlich, die Verbindungen I aus dem Reaktionsmedium zu isolieren. Sie können beispielsweise auch in Form der bei der Herstellung erhaltenen Lösungen angewendet werden. Zur Verbesserung der Stabilität können dem Reaktionsmedium vor, während oder nach der Reaktion Puffersubstanzen wie Phosphate, Maleinate Acetate, Carbonate oder Citrate zugesetzt werden.

2. 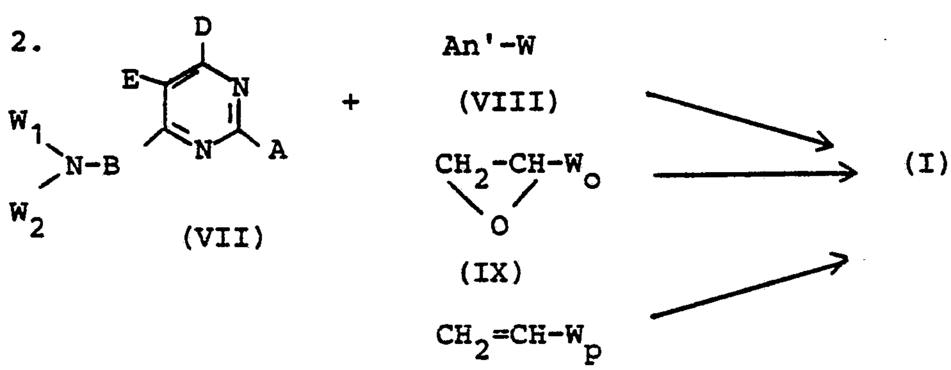

(VII) + An'-W (VIII) (IX) $CH_2$=CH-$W_p$ (X) (I)

wobei

An' für einen als Anion $An^{(-)}$ abspaltbaren Rest,
$W_o$ für Wasserstoff, Alkyl, das 2 Kohlenstoffatome weniger als W hat, Phenyl oder Phenoxymethyl,
$W_p$ für CN, $CO-NH_2$, $COO-R_5$ oder $SO_2R_5$ und
$R_5$ für Alkyl, Aryl oder Aralkyl stehen.

Als Beispiele für Verbindungen der Formel V seien aufgeführt:

1) 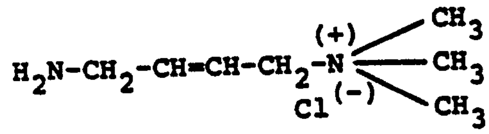

2) 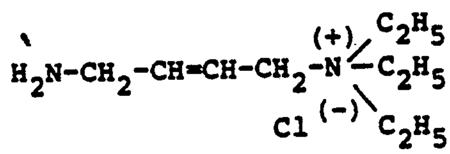

3) 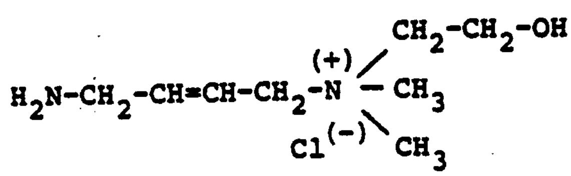

4) 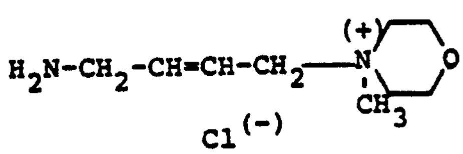

5) 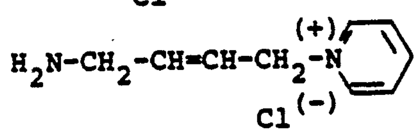

6) 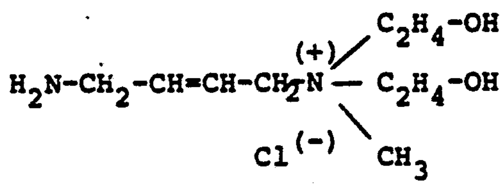

7) 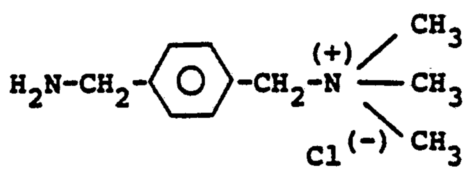

8) 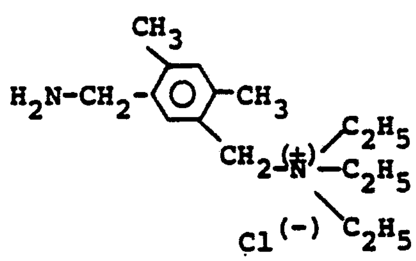

9) 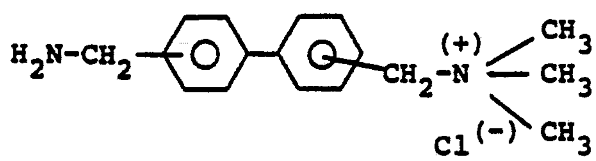

10) 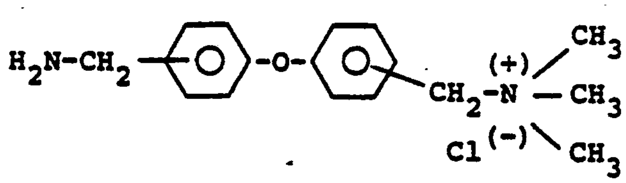

11) 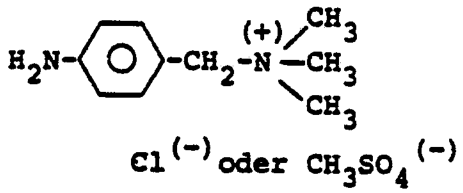

12) 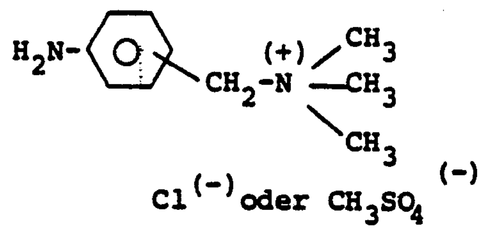

13) 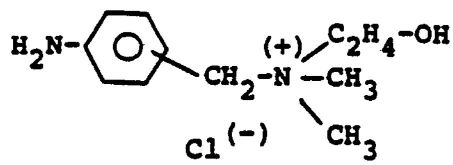

14) 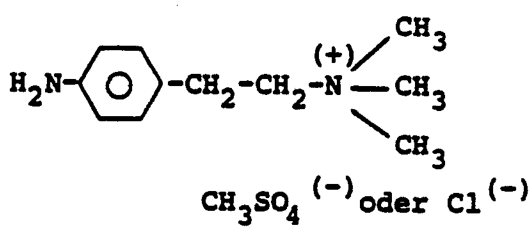

15) 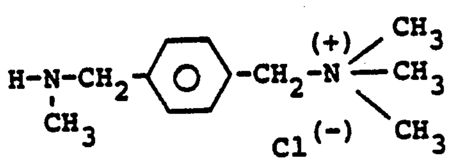

16) 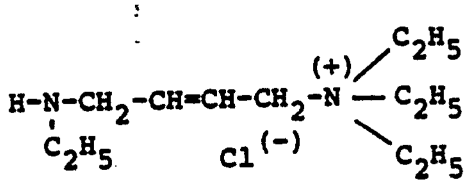

17) 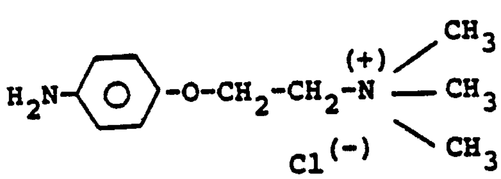

18) 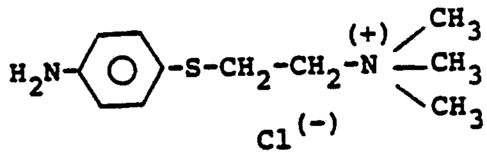

19) 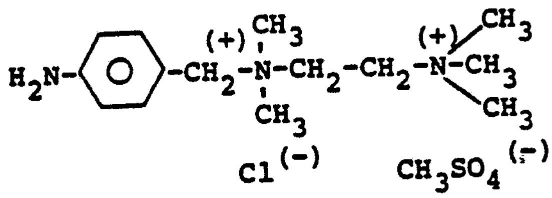

20) 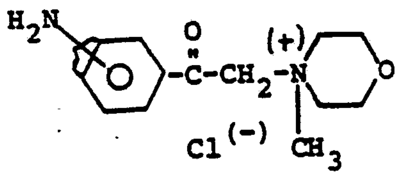

21) 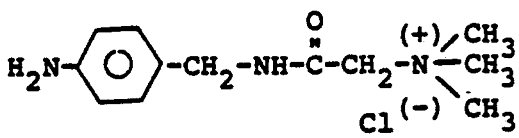

22) 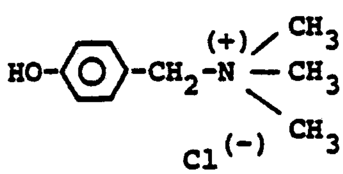

23) 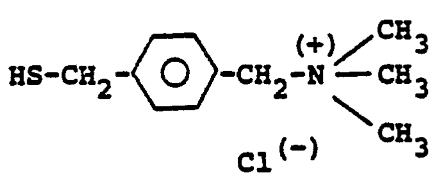

24) 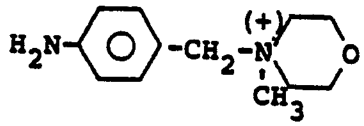

$CH_3-SO_4^{(-)}$

25) 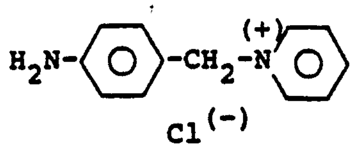

$Cl^{(-)}$

26) 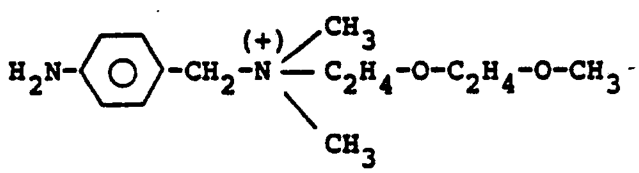

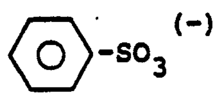

27) 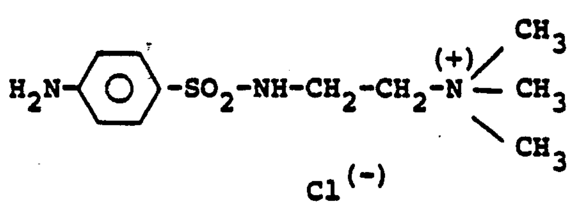

$Cl^{(-)}$

28) 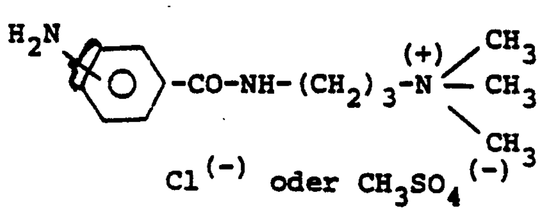

$Cl^{(-)}$ oder $CH_3SO_4^{(-)}$

Aus der großen Zahl der zur Verfügung stehenden Verbindungen VI seien hier auszugsweise erwähnt: Tetrahalogenpyrimidine, wie Tetrachlor-, Tetrabrom- oder Tetrafluor-pyrimidin, 2,4,6-Trihalogenpyrimidine, wie 2,4,6-Trichlor-, -Tribrom- oder -Trifluor-pyrimidin, Dihalogenpyrimidine, wie 2,4-Dichlor-, -Dibrom- oder -Difluorpyrimidin; 2,4,6-Trichlor-5-nitro- oder -5-methyl- oder -5-carbomethoxy- oder -5-carboäthoxy- oder -5-carboxymethyl- oder -5-mono-, -di- oder trichlormethyl- oder -5-carboxy- oder -5-sulfo- oder -5-cyano- oder -5-vinyl-pyrimidin, 2,4-Difluor-6-methylpyrimidin, 2,6-Difluor-4-methyl-5-chlorpyrimidin, 2,4-Difluor-pyrimidin-5-äthylsulfon, 2,6-Difluor-4-chlorpyrimidin, 2,4,6-Trifluor-5-chlorpyrimidin, 2,6-Difluor-4-methyl-5-brompyrimidin, 2,4-Difluor-5,6-dichlor- oder -dibrompyrimidin, 4,6-Difluor-2,5-dichlor- oder -dibrompyrimidin, 2,6-Difluor-4-brompyrimidin, 2,4,6-Trifluor-5-brompyrimidin, 2,4,6-Trifluor-5-chlormethylpyrimidin, 2,4,6-Trifluor-5-nitropyrimidin, 2,4,6-Trifluor-5-cyanpyrimidin, 2,4,6-Trifluorpyrimidin-5-carbonsäurealkylester oder -5-carbonsäureamide, 2,6-Difluor-5-methyl-4-chlorpyrimidin, 2,6-Difluor-5-chlorpyrimidin, 2,4,6-Trifluor-5-methylpyrimidin, 2,4,5-Trifluor-6-methylpyrimidin, 2,4-Difluor-5-nitro-6-chlorpyrimidin, 2,4-Difluor-5-cyanpyrimidin, 2,4-Difluor-5-methylpyrimidin, 6-Trifluormethyl-5-chlor-2,4-difluorpyrimidin, 6-Phenyl-2,4-difluorpyrimidin, 6-Trifluormethyl-2,4-difluorpyrimidin, 5-Trifluormethyl-2,4,6-trifluropyrimidin, 2,4-Difluor-5-nitro-pyrimidin, 2,4-Difluor-5-trifluormethyl-pyrimidin, 2,4-Difluor-5-methyl-

sulfonyl-pyrimidin, 2,4-Difluor-5-phenyl-pyrimidin, 2,4-Difluor-5-carbonamido-pyrimidin, 2,4-Difluor-5-carbomethoxy-pyrimidin, 2,4-Difluor-6-trifluormethyl-pyrimidin, 2,4-Difluor-5-brom-6-trifluormethyl-pyrimidin, 2,4-Difluor-6-carbonamido-pyrimidin, 2,4-Difluor-6-carbomethoxy-pyrimidin, 2,4-Difluor-6-phenyl-pyrimidin, 2,4-Difluor-6-cyan-pyrimidin, 2,4,6-Trifluor-5-methyl-sulfonyl-pyrimidin, 2,4-Difluor-5-sulfonamido-pyrimidin, 2,4-Difluor-5-chlor-6-carbomethoxy-pyrimidin,5-Trifluormethyl-2,4-difluorpyrimidin, Pyrimidin-Reaktivkomponenten mit abspaltbaren Sulfonylgruppen, wie 2-Carboxy-methyl-sulfonyl-4-chlorpyrimidin, 2-Methylsulfonyl-4-chlor-6-methylpyrimidin, 2,4-Bis-methylsulfonyl-6-methylpyrimidin, 2,4-Bis-phenylsulfonyl-5-chlor-6-methylpyrimidin, 2,4,6-Tris-methylsulfonylpyrimidin, 2,6-Bismethylsulfonyl-4,5-dichlorpyrimidin, 2-Methylsulfonyl-4-chlorpyrimidin, 2-Phenylsulfonyl-4-chlorpyrimidin, 2,4-Bis-trichlormethyl-sulfonyl-6-methylpyrimidin, 2,4-Bis-methylsulfonyl-5-chlor-6-methylpyrimidin, 2,4-Bis-methylsulfonyl-5-brom-6-methyl-pyrimidin, 2-Methylsulfonyl-4,5-dichlor-6-methylpyrimidin, 2-Methylsulfonyl-4,5-dichlor-6-chlor-methylpyrimidin, 2-Methylsulfonyl-4-chlor-5-nitro-6-methylpyrimidin, 2,4,5,6-Tetramethylsulfonyl-pyrimidin, 2-Methylsulfonyl-4-chlor-5,6-dimethylpyrimidin, 2-Äthylsulfonyl-4,5-dichlor-6-methylpyrimidin, 2-Methylsulfonyl-4,6-dichlorpyrimidin, 2,4,6-Tris-methylsulfonyl-5-chlor-pyrimidin, 2-Methylsulfonyl-4-chlor-6-carboxypyrimidin, 2-Methylsulfonyl-4-chlor-6-carbomethoxypyrimidin, 2-Methylsulfonyl-4-chlor-5-cyan-6-methoxypyrimidin, 2-Methylsulfonyl-4,5-dichlorpyrimidin, 4,6-Bis-methylsulfonylpyrimidin, 4-Methylsulfonyl-6-chlorpyrimidin, 2-Sulfoäthylsulfonyl-4-chlor-6-methylpyrimidin, 2-Methylsulfonyl-4-chlor-5-brompyrimidin, 2-Methylsulfonyl-4-chlorbrom-6-methylpyrimidin, 2,4-Bis-methylsulfonyl-5-chlorpyrimidin, 2-Phenylsulfonyl-4,5-dichlorpyrimidin, 2-Phenylsulfonyl-4,5-dichlor-6-methylpyrimidin, 2-Carboxymethylsulfonyl-4,5-dichlor-6-methylpyrimidin, 2-(2'- oder 3'- oder 4'-Carboxyphenylsulfonyl)-4,5-dichlor-6-methylpyrimidin, 2,4-bis-(2'- oder 3'- oder 4'-Carboxyphenylsulfonyl)-5-chlor-6-methylpyrimidin.

Als Quarternierungsmittel (VIII), (IX) und (X) seien genannt: Alkylhalogenide, Aralkylhalogenide, Cycloalkylhalogenide, Dialkylsulfate, Alkylester von Arylsulfonsäuren sowie andere Ester starker Mineralsäuren und organischer Sulfonsäuren von vorzugsweise niederen Alkoholen. In Gegenwart von Säuren können auch Acrylsäure und ihre Derivat und Epoxide als Alkylierungsmittel verwendet werden. Die quarternierenden Mittel können weiter substituiert sein. Beispiele sind: Dimethylsulfat, Diäthylsulfat, Methylchlorid, Methylbromid, Methyljodid, Methylsulfat, Äthylbromid, n-Propylbromid, n-Butylbromid, Allylchlorid, Chlor- und Bromessigsäuremethylester, Methansulfonsäuremethyl- und -äthylester, Äthylenchlorhydrin, Chloracetonitril, Benzylchlorid, Phenyläthylchlorid, Phenoxy-ß-chloräthyl, Butenylchlorid, Chloramin, O-Methylsulfonylhydroxylamin, O-Mesitylensulfonylhydroxylamin, N,N-Dimethylchloramin, Hydroxylamin-O-sulfonsäure, Acrylnitril, Äthylenoxid und Propylenoxid.

Man kann die erfindungsgemäß zu verwendenden Verbindungen der Formel (I) in einem Vorbehandlungsprozeß auf das zu färbende Fasermaterial aufbringen, oder man wendet sie einbadig mit den anionischen Farbstoffen zusammen an. Man kann sie auch in einem Nachbehandlungsprozeß auf fertige Färbung und Drucke aufbringen. Im Falle einer Vorbehandlung wird das mit den Verbindungen der Formel (I) behandelte Fasermaterial anschließend aus langer Flotte in der für die jeweiligen Farbstoffe üblichen Weise gefärbt. Man kann das Material aber auch aus kurzer Flotte färben.

Eine Vorbehandlung erfolgt durch Imprägnieren oder Bedrucken mit wäßrigen Klotzflotten bzw. Druckpasten, die neben den Verbindungen (I) in Mengen von 20 - 150 g, vorzugsweise 40 - 100 g, pro Liter Klotzflotte bzw. Druckpaste das zur chemischen Umsetzung mit dem Fasermaterial notwendige Alkali, z.B. Natriumbicarbonat, Natriumhydroxid oder vorzugsweise Natriumcarbonat, enthält. Das so imprägnierte Fasermaterial wird auf eine Flottenaufnahme von 60 - 100 %, vorzugsweise 70 - 90 %, des Fasergewichts abgepreßt und mit oder ohne Zwischentrocknung einer Hitzebehandlung unterzogen; dies kann durch kurzfristiges Dämpfen, z.B. 3 bis 10 Minuten Dampfbehandlung bei 102 bis 120°C, oder durch kurzfristige Trockenhitzebehandlung, z.B. 2 bis 10 Minuten bei 120 bis 150°C erfolgen; bei dieser Hitzebehandlung erfolgt die Reaktion der Verbindungen (I) mit dem Fasermaterial unter Ausbildung einer chemischen Bindung. Die Reaktion mit dem

Fasermaterial kann aber auch in einem Kaltverweilverfahren erfolgen, indem man das imprägnierte und abgepreßte Material aufrollt und bei Zimmertemperatur 4 bis 24 Stunden bzw. bei Raumtemperatur aufbewahrt. Durch Umwickeln mit wasserundurchlässigem Material muß dabei Sorge getragen werden, daß kein Wasser verdampfen kann.

Die Verweilzeit kann durch Erhöhung der Verweiltemperatur von Raumtemperatur auf 60 - 80°C auf eine bis vier Stunden vermindert werden.

Bei der einbadigen Anwendung der Verbindungen (I) zusammen mit Farbstoffen, stellt man Klotzflotten bzw. Druckpasten her, die neben den Verbindungen (I) in Mengen von 20 - 150 g, vorzugsweise 40 - 100 g je Liter Klotzflotte bzw. je kg Druckpaste, die anionischen Farbstoffe und noch die in der Färberei üblichen Klotzhilfsmittel und Netzmittel, die im Textildruck üblichen Verdickungsmittel und Reduktionsmittel sowie das notwendige alkalische Mittel, z.B. Natriumbicarbonat, Natriumcarbonat oder Natriumhydroxid enthalten. Die anschließende Fixierung erfolgt in der üblichen Weise durch eine Dampf-, Trockenhitze- oder Kaltverweilbehandlung.

Zur Nachbehandlung fertiger Färbungen oder Drucke setzt man die Verbindungen (I) in Mengen von 0,1 - 16 %, vorzugsweise 1 - 12 %, bezogen auf das Substrat in

wäßriger Lösung ein. Die Lösungen enthalten 1 - 5 ml/l Natriumhydroxid (50 %ig). In diesen Lösungen werden die fertigen Färbungen 10 - 60 Minuten lang bei Temperaturen von 20 - 80°C, vorzugsweise 20 - 40°C, behandelt.

Zur Nachbehandlung kann man auch Klotzflotten herstellen, die neben den Verbindungen der Zusammensetzung (I) in Mengen von 20 - 150 g, vorzugsweise 40 - 100 g je Liter Klotzflotte, noch die in der Färberei üblichen Klotzhilfsmittel und Netzmittel sowie das notwendige alkalische Mittel, z.B. Natriumbicarbonat, Natriumcarbonat oder Natriumhydroxid enthalten. Hier erfolgt die anschließende Fixierung durch eine Dampf-, Trockenhitze- oder Kaltverweilbehandlung.

Mit Hilfe der erfindungsgemäßen Verbindungen (I) erzielt man eine überraschend große Verbesserung der Farbechtheiten, insbesondere der Wasser-, Schweiß- und Waschechtheiten, gegenüber den Farbechtheiten üblicher Färbungen und Drucke mit anionischen Farbstoffen. Es werden außerdem tiefere Färbungen und Drucke erzielt, da Spül- und Seifnachbehandlung zu keiner Ablösung der Farbstoffe vom Fasermaterial führen, wie es sonst bei nicht behandelten Färbungen und Drucken der Fall ist.

Die Verwendung der Verbindungen (I) erfolgt auf Textilmaterial aus natürlicher Cellulose, wie Baumwolle oder Leinen, oder regenerierte Cellulose wie Zellwolle,

Rayon oder Modulfasern; diese Fasermaterialien können sowohl allein als auch in Mischungen mit synthetischen Fasermaterialien z.B. solchen aus Polyester, Polyamid oder Polyacrylnitril vorliegen.

Verbindungen der Zusammensetzung (I) können zur Vor-, Einbad- und Nachbehandlung von Färbungen und Drucken mit solchen Farbstoffen dienen, wie sie z.B. im Colour Index, 3. Auflage (1971), Bd. 1 auf den Seiten 1001 - 1561 als saure Farbstoffe und Bd. 2 auf den Seiten 2005 - 2477 als Direktfarbstoffe aufgeführt sind.

Im Vergleich zu den bekannten kationaktiven Nachbehandlungsmitteln für Direktfarbstoffe bestehen Vorteile der Verbindungen der Zusammensetzung (I) darin, daß die mit ihnen vor-, einbadig- bzw. nachbehandelten Färbungen bedeutend verbesserte Waschechtheiten auch bei erhöhten Waschtemperaturen, z.B. bei 60°C, und sogar bei 95°C, ergeben. Die Lichtechtheiten werden nicht negativ beeinflußt, und es sind nur geringfügige oder keine Farbtonänderungen feststellbar.

Beispiel 1

Trimethyl-(4-(3)-aminobenzyl)-ammonium-methosulfat (Mischung)

Zu einer Lösung von 150 g (1 Mol) einer technischen Mischung von Dimethyl-(4- und 3-aminobenzyl)-amin in 1000 ml Aceton läßt man bei 20-30°C 126 g (1 Mol) Dimethylsulfat zutropfen. Man saugt das ausgefallene, farblose Reaktionsprodukt ab und erhält nach Trocknen im Vakuum ca. 262 g (95 % d.Th.) einer Mischung der Verbindungen der Formeln

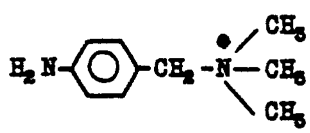 $CH_3OSO_3^{\ominus}$

und

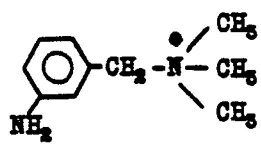 $CH_3OSO_3^{\ominus}$

Beispiel 2

Trimethyl-(3-aminobenzyl)-ammonium-methosulfat

Zu einer Lösung von 150 g (1 Mol) Dimethyl-(3-aminobenzyl)-amin in 1000 ml Aceton läßt man bei 20-30°C 126 g (1 Mol) Dimethylsulfat zutropfen. Man saugt das ausgefallene, farblose Reaktionsprodukt ab und erhält nach Trocknen im Vakuum ca. 260 g (94 % d.Th.) der Verbindung der Formel

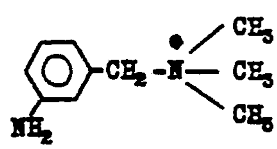 $CH_3OSO_3^{\ominus}$

(Schmelzpunkt: 145°C). Die Verbindung kann aus Isopropanol umkristallisiert werden.

Synthese der reaktiven Salze der Formel I

Beispiel 3

Zu einer Lösung von 276 g (1 Mol) Trimethyl-(4-(3)-amino-benzyl)-ammonium-methosulfat (Mischung) in 800 ml Wasser werden bei 20°C 168,5 g (1 Mol) 2,4,6-Trifluor-5-chlor-pyrimidin unter Rühren zugetropft. Durch Zugabe von Natronlauge, Sodalösung oder Bicarbonat wird ein pH-Wert von 6 eingehalten. Anschließend rührt man noch ca. 4 Stunden nach, wobei der pH-Wert mit Natronlauge, Sodalösung oder Bicarbonat bei 7 gehalten wird und filtriert gegebenenfalls. Man erhält eine Lösung, die ca. 390-420 g einer Mischung der Verbindungen der Formeln

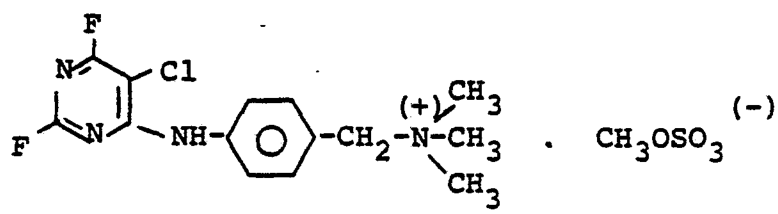

und

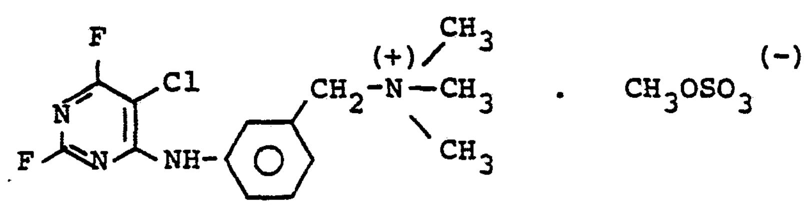

enthält. Die Charakterisierung erfolgte durch NMR-Spektrum. Man kann die wäßrige Lösung bei 20°C im Vakuum einengen und erhält eine Paste, welche die Mischung dieser Verbindungen enthält; man kann aber auch die wäßrige Lösung direkt verwenden, oder aber die Mischung der Verbindungen aussalzen. Fp. der Mischung: 173-176°C.

Beispiel 4

Zu einer Lösung einer technischen Mischung von 150 g (1 Mol) Dimethyl-(4- und 3-aminobenzyl)-amin in 800 ml Wasser läßt man unter Rühren bei 0-20°C 126 g (1 Mol) Dimethylsulfat zutropfen. Nach Abklingen der Wärmetönung rührt man noch 2 Stunden und versetzt anschließend bei ca. 50°C mit 164,5 g (1 Mol) 2,4-Difluor-5-chlor-6-methyl-pyrimidin, wobei durch Zugage von Natronlauge, Bicarbonat oder Sodalösung ein pH-Wert von 6 eingehalten wird. Anschließend rührt man noch ca. 4 Stunden nach, wobei der pH-Wert mit Natronlauge, Bicarbonat oder Sodalösung bei 7 gehalten wird und filtriert gegebenenfalls. Die erhaltene Lösung enthält ca. 375-415 g einer Mischung der Verbindungen der Formeln

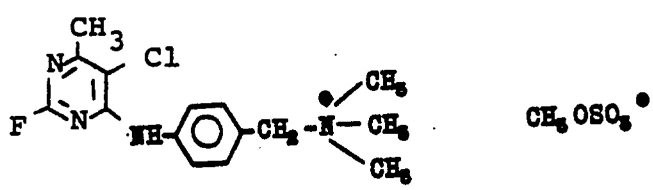

und

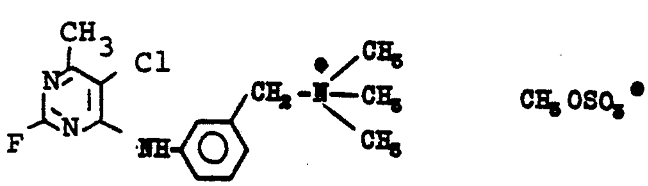

Die Lösung kann direkt in dieser Form verwendet werden.

Beispiel 5

Zu einer Lösung von 276 g (1 Mol) Trimethyl-(3-amino-benzyl)-ammonium-methosulfat in 1000 ml Wasser werden bei 20-30$^{o}$C unter Rühren 241 g (1 Mol) 2-Methylsulfonyl-4,5-dichlor-6-methyl-pyrimidin zugegeben und anschließend wird auf 60$^{o}$C erhitzt. Durch Zugabe von Natronlauge, Bicarbonat oder Sodalösung wird ein pH-Wert von 6 eingehalten. Man läßt dann bis zur klaren Lösung rühren, wobei der pH-Wert mit Natronlauge, Bicarbonat oder Sodalösung bei 7 gehalten wird und filtriert gegebenenfalls. Man erhält eine Lösung, die ca. 450-475 g der Verbindung der Formel

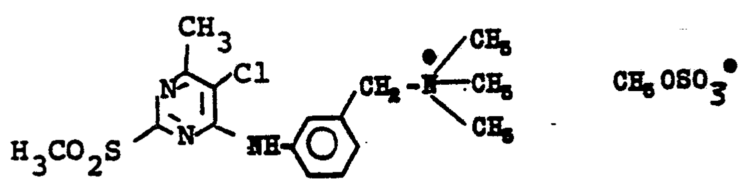

enthält. Diese Lösung kann man direkt verwenden, oder je nach Bedarf verdünnen, oder durch Absiehen von Wasser im Vakuum bei 20-30°C aufkonzentrieren. Dasselbe gilt z.B. ebenso für die nach den Beispielen 3 und 4 erhaltenen Lösungen.

Man kann aber auch z.B. die nach diesem und nach den Beispielen 3 und 4 erhaltenen quartären Reaktiv-Verbindungen z.B. in der Weise isolieren, daß man das Wasser im Vakuum bei 20-30°C abzieht, den Rückstand mit Methanol eluiert, von den ungelösten anorganischen Salzen abfiltriert und die methanolische Lösung einengt.

Einfacher und ökonomischer ist es hingegen, die erhaltene, gegebenenfalls, wie oben beschrieben, verdünnte oder aufkonzentrierte wässrige Lösung direkt zu verwenden.

In der folgenden Tabelle sind weitere Beispiele von Verbindungen der allgemeinen Formel I aufgeführt, die man erhalten kann, wenn man analog wie in den Beispielen 3 bis 5 verfährt.

| Bsp. Nr. | Verbindung V | Verbindung VI | Verbindung I |
|---|---|---|---|
| 6 | 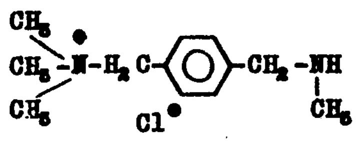 | 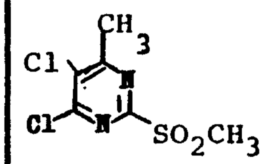 | 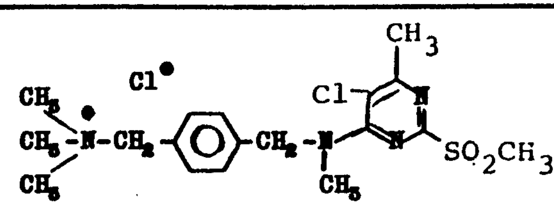 |
| 7 | 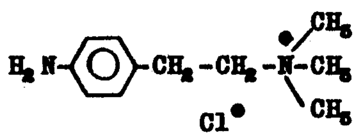 | 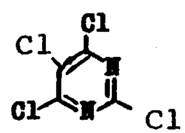 | 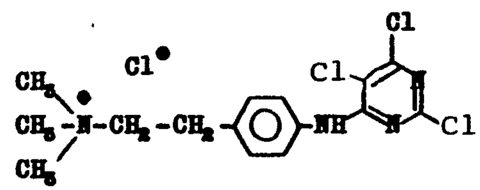 |
| 8 | 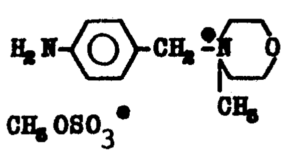 | 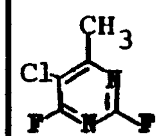 | 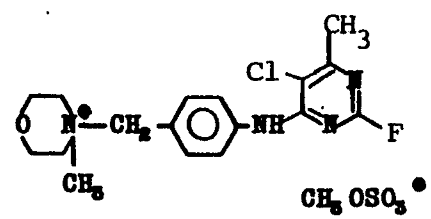 |
| 9 | 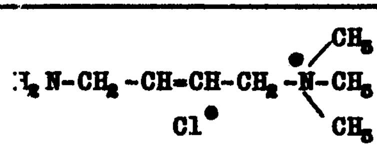 | 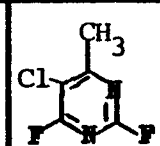 | 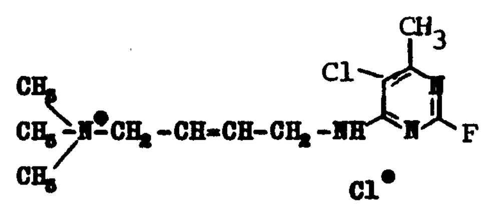 |

| Bsp. Nr. | Verbindung V | Verbindung VI |
|---|---|---|
| 10 | 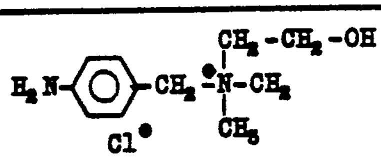 | 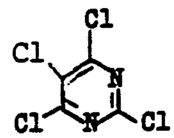 |
| 11 | 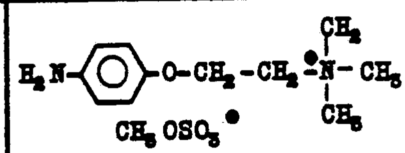 | 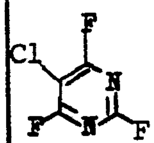 |
| 12 | 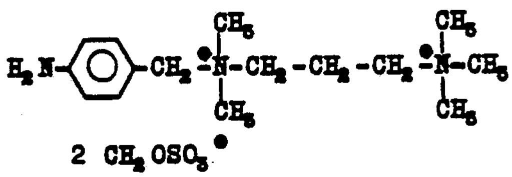 | 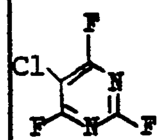 |
| 13 | 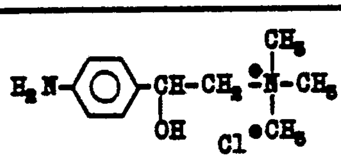 | 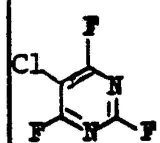 |
| 14 | 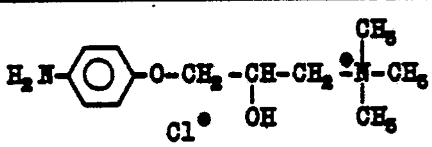 | 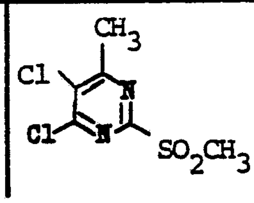 |

| Bsp. Nr. | Verbindung I |
|---|---|
| 10 | 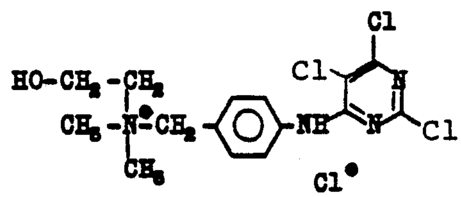 |
| 11 | 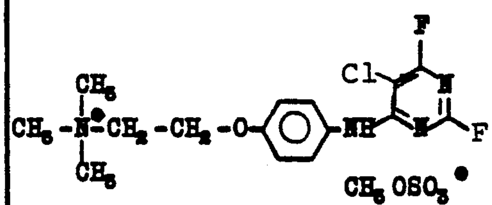 |
| 12 | 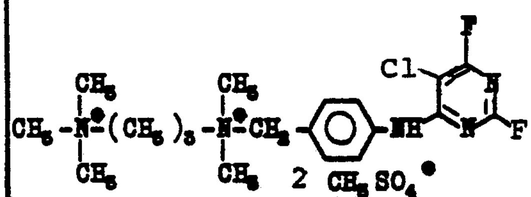 |
| 13 | 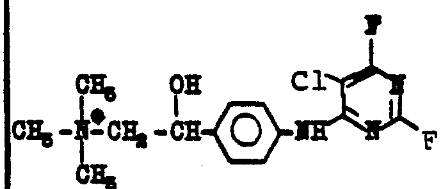 $Cl^{\ominus}$ |
| 14 | 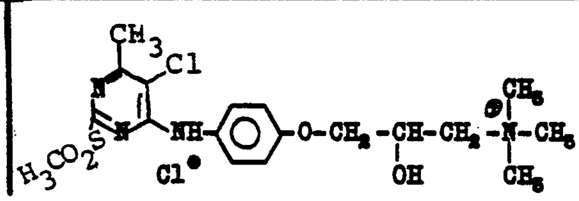 |

Beispiel 15

Auf einem Baumwollgewebeabschnitt wird eine Färbung von 3 % C.I. Direct Blue 225 (Colour Index (1971) 3. Aufl. Bd. 2) in der für diese Farbstoffe üblichen Färbeweise aus wäßriger Färbeflotte hergestellt, gespült und getrocknet. Auf einem Färbefoulard imprägniert man diese Färbung bei Zimmertemperatur mit einer Lösung, die im Liter

100 g der Verbindungen des Beispiels 3
20 g Natriumcarbonat enthält,

und so abgepreßt wird, daß die Flottenaufnahme der Färbung etwa 80 % beträgt. Das Material wird sodann im Trockenschrank bei 60-70 °C getrocknet und anschließend 3 Minuten lang bei 150 °C gedämpft. Beim nun nachließenden Spülen bei Raumtemperatur und 60-70 °C während jeweils 5 Minuten bleiben die Spülbäder ungefärbt, beim darauffolgenden Behandeln im destillierten Wasser bei 95 °C erfolgt innerhalb 20 Minuten ein nur geringfügiges Anfärben der Behandlungsflotte.

Man erhält eine Blaufärbung mit sehr guten Licht-, Wasser-, Schweiß- und Waschechtheiten.

Eine Blaufärbung mit den genannten vorteilhaften Eigenschaften erhält man auch, wenn man als Farbstoff C.I. Direct Blue 151 (=C.I. No. 24175) verwendet.

Beispiel 16

Einen Baumwollgewebeabschnitt, der mit 3 % C.I. Direct Blue 225 gefärbt ist, behandelt man in einem frischen Bad, das - bezogen auf das Gewicht der Baumwolle -

8 % der Verbindungen der Beispiele 3 bzw. 4 sowie
2 ml/l·50 %ige Natriumhydroxidlösung enthält,

im Flottenverhältnis 1:30 bei 20-25°C innerhalb von 25 Minuten. Dann spült man das Gewebe in kaltem und 60°C heißem Wasser aus und trocknet das Baumwollgewebe. Man erhält eine Blaufärbung mit sehr guter Licht-, Wasser-, Schweiß- und Waschechtheit.

Naheliegende Ergebnisse erhält man, wenn man z.B. anstelle von
8 % der Verbindungen der Beispiele 3 bzw. 4
8 % der Verbindung des Beispiels 8
10 % der Verbindung des Beispiels 11
10 % der Verbindung des Beispiels 13
12 % der Verbindung des Beispiels 14
verwendet.

Beispiel 17

Man stellt eine wässrige Lösung her, die im Liter

60 g der Verbindung des Beispiels 5
30 g des Farbstoffs C.I. Direct Orange 76 (=C.I.No. 40270)

Zu dieser Lösung gibt man 15 g Natriumcarbonat, imprägniert einen Baumwollgewebeabschnitt mit der Lösung und preßt ihn so ab, daß eine Flottenaufnahme von 80 % erfolgt. Das so behandelte Gewebe wird bei 60°C getrocknet und 3 Minuten lang auf 150°C erhitzt. Beim anschließenden Ausspülen in Wasser von Raumtemperatur und 60°C während jeweils 5 Minuten und beim Behandeln in siedendem Wasser während 20 Minuten erfolgt kein Anfärben der Behandlungsflotten.

Die so erhaltene Scharlachfärbung zeichnet sich durch gute Licht-, Wasser-, Schweiß- und Waschechtheit aus.

Naheliegende Ergebnisse erhält man, wenn man anstelle von
60 g der Verbindung des Beispiels 5
80 g der Verbindung des Beispiels 6

80 g der Verbindung des Beispiels 7
70 g der Verbindung des Beispiels 8
80 g der Verbindung des Beispiels 14
verwendet.

Patentansprüche

1. Verbindungen der Formel

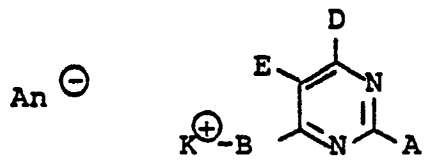

worin

A ein reaktionsfähiger, abspaltbarer Substituent,

E Wasserstoff, Halogen, gegebenenfalls substituiertes Alkyl, Aryl, Cyano, Aminocarbonyl, Mono- oder Dialkylaminocarbonyl, Alkoxycarbonyl, Nitro, gegebenenfalls substituiertes Alkyl-, Aryl- oder Aralkylsulfonyl,

D Wasserstoff, Halogen, gegebenenfalls substituiertes Alkyl, Aryl, Aralkyl, Aminocarbonyl, Mono- oder Dialkylaminocarbonyl, Hydroxy, gegebenenfalls substituiertes Alkoxy, Aryloxy, Aralkoxy, Alkylthio, Arylthio, Aralkylthio, gegebenenfalls substituiertes Alkyl-, Aryl- oder Aralkylsulfonyl oder einen Rest der Formel

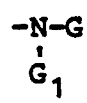

G und $G_1$ unabhängig voneinander Wasserstoff, gegebenenfalls substituiertes Alkyl, Aryl, Aralkyl oder gemeinsam mit N einen 5- oder 6-gliedrigen Ring bedeuten, worin

A auch für Wasserstoff oder einen nichtreaktionsfähigen Substituenten stehen kann, wenn

D für Halogen oder gegebenenfalls substituiertes Alkyl, Aryl- oder Aralkylsulfonyl steht, und worin

B ein Bindeglied

$- X - Y - Z - Y_1 -$

bedeutet,
wobei X mit dem Diazinrest verbunden ist,

X Sauerstoff, Schwefel oder 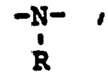

R Wasserstoff, gegebenenfalls substituiertes Alkyl, Alkenyl oder Aralkyl,

Y 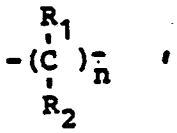

n die Zahlen 0 bis 4

Z einen gegebenenfalls substituierten Phenylen- oder Naphthylen-Rest oder den Rest der Formel $-CH=CH-$,

$Y_1$ einen Rest der Formeln

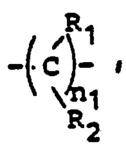 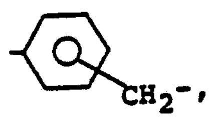 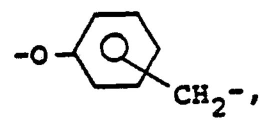 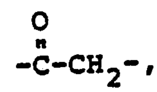

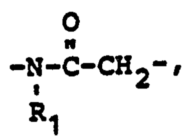 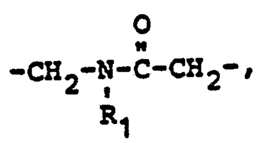 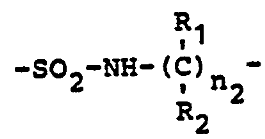

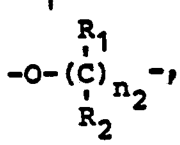 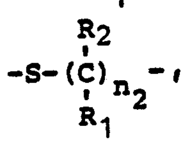 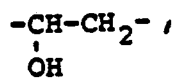

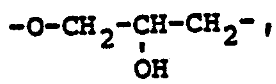

$n_1$ die Zahlen 0 bis 4, wobei n und $n_1$ nicht gleichzeitig 0 sind,

$n_2$ die Zahlen 2 bis 5,

$R_1$ und $R_2$ unabhängig voneinander Wasserstoff oder Alkyl,

$K^{(+)}$ einen quartären Rest

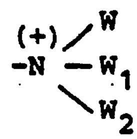

$W, W_1$ und $W_2$ unabhängig voneinander gegebenenfalls substituiertes Alkyl, Cycloalkyl, Alkenyl oder Aralkyl,

$W_1$ und $W_2$ oder

$W, W_1$ und $W_2$ gemeinsam mit dem Stickstoffatom einen gegebenenfalls substituierten Heterocyclus oder

W einen Amino-, Monoalkyl- oder Dialkylamino-Rest oder einen Rest der Formel

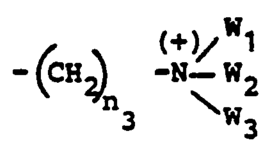

$n_3$ die Zahlen 2 bis 4,

$W_3$ einen gegebenenfalls substituierten Alkylrest und

$An^{(-)}$ ein Anion bedeuten.

2. Verbindungen der Formel

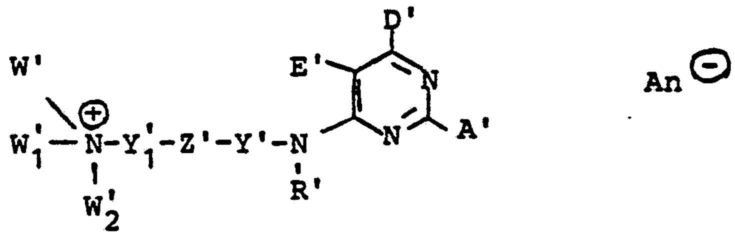

worin

A' für Fluor, Chlor, Methyl-, Ethyl- oder Phenylsulfonyl,

E' für Wasserstoff, Fluor, Chlor, Methyl, Ethyl, Phenyl, Cyano, Nitro oder Methyl-, Ethyl- oder Phenylsulfonyl

D' für Wasserstoff, Fluor, Chlor, Methyl, Ethyl, Methyl-, Ethyl- oder Phenylsulfonyl, gegebenenfalls durch Fluor, Chlor, Brom, Cyan oder Hydroxy substituiertes $C_1$-$C_4$-Alkyl, $C_1$-$C_4$-Alkoxy oder $C_1$-$C_4$-Alkylthio oder für einen Rest

$$-\underset{G_1'}{\overset{|}{N}}-G'$$

mit G' und $G_1'$ = Wasserstoff, gegebenenfalls durch Hydroxyl substituiertes $C_1$-$C_4$-Alkyl, Phenyl oder Benzyl, oder für

$$-(CH_2)_{n_4}-(O-\overset{R_3}{\overset{|}{CH}}-\overset{R_3}{\overset{|}{CH}})_{n_4}-O-R_4'$$

mit $n_4$ = unabhängig voneinander = 0 bis 4
$R_3$ = beide Wasserstoff oder 1 $R_3$ Wasserstoff und das andere Methyl,
$R_4'$ = Wasserstoff oder $C_1$-$C_4$-Alkyl,

stehen, worin

A' auch für Wasserstoff stehen kann, wenn
D' für Fluor, Chlor, Methyl-, Ethyl- oder Phenylsulfonyl steht, und worin
R' für Wasserstoff oder $C_1$-$C_4$-Alkyl, das durch

$$-(O-\overset{R_3}{\overset{|}{C}}H-\overset{R_3}{\overset{|}{C}}H)_{n_4}-O-R_4$$

substituiert sein kann,

Y' für $-\left(\begin{array}{c} R_1' \\ | \\ C \\ | \\ R_2' \end{array}\right)_{n'}-$,

mit $R_1'$ und $R_2'$ = Wasserstoff oder $C_1$-$C_4$-Alkyl,
$n'$ = 0 oder 1,

Z' für gegebenenfalls durch Fluor, Chlor, Brom, Hydroxy, $C_1$-$C_4$-Alkyl oder $C_1$-$C_4$-Alkoxy substituiertes Phenylen oder Naphthylen,
$Y_1'$ für einen Rest der Formeln

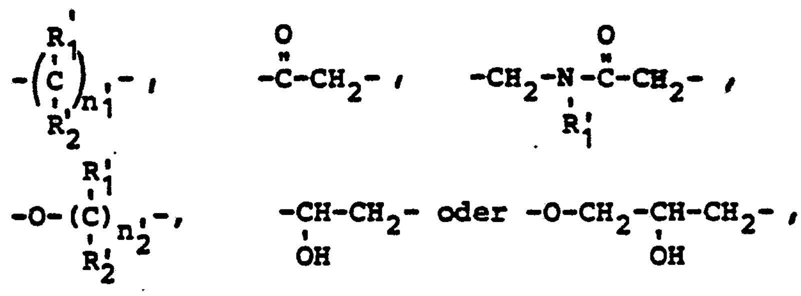

mit

$n_1' = 1$ oder 2,

$n_2' = 2$ oder 3,

W', $W_1'$ und $W_2'$ unabhängig voneinander für $C_1$-$C_6$-Alkyl, das durch

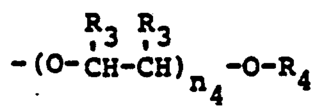

substituiert sein kann,

W' außerdem für Benzyl, Amino, $C_1$-$C_4$-Alkylamino oder $C_1$-$C_4$-Dialkylamino oder

$W_1'$ und $W_2'$ gemeinsam mit dem Stickstoffatom für Pyrrolidin, Piperidin, Morpholin oder Piperazin oder

W', $W_1'$ und $W_2'$ gemeinsam mit dem Stickstoffatom für Pyridin und

$An^{(-)}$ für ein Anion stehen.

3. Verbindungen nach Anspruch 2, dadurch gekennzeichnet, daß

n' null,

Z' gegebenenfalls durch Methyl substituiertes Phenylen und

$Y_1'$ Methylen oder Ethylen

bedeuten.

4. Verbindungen der Formel

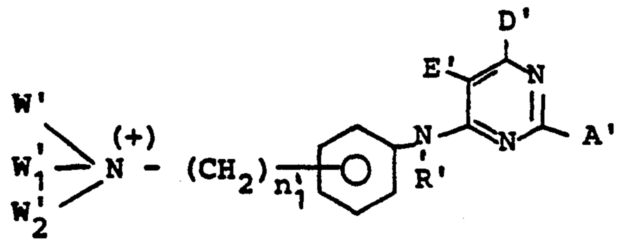

$An^{(-)}$

worin

A', D', E', R', W', $W_1$', $W_2$', $n_1$' und $An^{(-)}$ die in Anspruch 2 angegebene Bedeutung haben.

5. Verbindungen nach Anspruch 4, dadurch gekennzeichnet, daß

R' Wasserstoff oder Methyl,

W', $W_1$' und $W_2$' Methyl, Äthyl oder Hydroxyäthyl und

$n_1$' 1 bedeuten.

6. Verfahren zur Herstellung von Verbindungen des Anspruchs 1, dadurch gekennzeichnet, daß man eine Verbindung der Formel

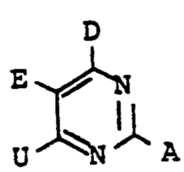

mit einer Verbindung der Formel

$$K^{(+)}-B-H \cdot An^{(-)}$$

umsetzt, worin

D, E, A, $K^{(+)}$, B und $An^{(-)}$ die in Anspruch 1 angegebene Bedeutung haben, und

U einen Rest bedeutet, der unter den Reaktionsbedingungen abpaltet unter Bildung von HU.

7. Verfahren zur Herstellung von Verbindungen des Anspruchs 1, dadurch gekennzeichnet, daß man eine Verbindung der Formel

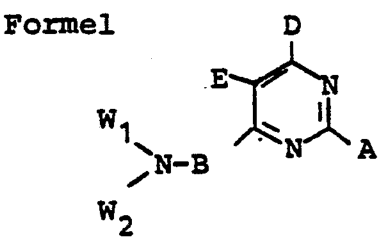

mit einer Verbindung der Formel

$$An'-W$$

oder einer Verbindung der Formel

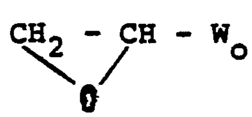

oder einer Verbindung der Formel

$$CH_2 = CH - W_p$$

umsetzt, worin

| | |
|---|---|
| A,D,E,B,W,$W_1$ und $W_2$ | die in Anspruch 1 angegebene Bedeutung haben, und |
| An' | für einen als Anion $An^{(-)}$ abspaltbaren Rest, |
| $W_o$ | für Wasserstoff, Alkyl, das 2 Kohlenstoffatome weniger als W hat, für Phenyl oder Phenoxymethyl, |
| $W_p$ | für CN, $CO-NH_2$, $COOR_5$ oder $SO_2-R_5$ und |
| $R_5$ | für Alkyl, Aryl oder Aralkyl stehen. |

8. Verfahren zur Erhöhung der Affinität von anionischen Farbstoffen zu natürlichen oder synthetischen stickstoff- oder hydroxylgruppenhaltigen Fasern, dadurch gekennzeichnet, daß man Verbindungen des Anspruchs 1 verwendet.

0015365

Nummer der Anmeldung

EP 80 10 0205

Europäisches Patentamt

# EUROPÄISCHER RECHERCHENBERICHT

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| X | FR - A - 2 354 320 (BAYER)<br>* Seiten 22,23,27,34-39 *<br>-- | 1-4,6, 8 | C 07 D 239/42<br>239/46<br>239/34<br>239/38<br>239/56<br>D 06 P 1/66 |
| X | DE - A - 2 726 432 (CIBA-GEIGY)<br>* Seiten 1-9,25; Beispiel 7 *<br>---- | 1-4,6, 8 | |
| | | | RECHERCHIERTE SACHGEBIETE (Int. Cl. 3) |
| | | | C 07 D 239/42<br>239/46<br>239/34<br>239/38<br>239/56<br>D 06 P 1/66 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 16-05-1980 | FRANCOIS |